(19) **Europäisches Patentamt / European Patent Office / Office européen des brevets**

(11) **EP 4 596 704 A1**

(12) **EUROPEAN PATENT APPLICATION**
published in accordance with Art. 153(4) EPC

(43) Date of publication:
06.08.2025 Bulletin 2025/32

(21) Application number: 23872593.1

(22) Date of filing: 29.09.2023

(51) International Patent Classification (IPC):
*C12P 21/00* (2006.01)   *C12P 21/08* (2006.01)

(52) Cooperative Patent Classification (CPC):
C07K 16/00; C12P 21/00

(86) International application number:
PCT/JP2023/035594

(87) International publication number:
WO 2024/071373 (04.04.2024 Gazette 2024/14)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 29.09.2022  JP 2022156714

(71) Applicant: FUJIFILM Corporation
Tokyo 106-8620 (JP)

(72) Inventors:
• KUROSAWA Takashi
  Ashigarakami-gun, Kanagawa 258-8577 (JP)
• NAKAI Shinichi
  Ashigarakami-gun, Kanagawa 258-8577 (JP)
• INADA Atsushi
  Ashigarakami-gun, Kanagawa 258-8577 (JP)

(74) Representative: Hoffmann Eitle
Patent- und Rechtsanwälte PartmbB
Arabellastraße 30
81925 München (DE)

(54) **METHOD FOR PRODUCING PRODUCT**

(57) An object of the present invention is to provide a method that makes it possible to suppress membrane clogging (primary clogging) in a production method for a product by cell culture. According to the present invention, there is provided a production method for a product, which includes culturing cells in a culture tank, in which a cell density at a time of production of the product is $80 \times 10^6$ cells/mL or more and $300 \times 10^6$ cells/mL or less, the culturing is perfusion culture, a culture scale is 5 L or more and 100,000 L or less, a cumulative number X [particles/mm$^2$] of particles having a particle diameter of 1.47 to 6.00 μm on Day 10 at the time of the production of the product per surface area of a membrane for separating cells and a product in a culture solution satisfies $0 < X \le 1.0 \times 10^8$, and an aeration rate V [vvm] satisfies $0.09 \le V \le 1.0$.

EP 4 596 704 A1

## Description

Technical Field

The present invention relates to a production method for a product, which includes culturing cells, in which a cumulative number of particles per surface area of a membrane that separates the cells and the product, and an aeration rate are controlled.

Background Art

Cell culture is carried out for the intended purpose, for example, increasing the number of cells having useful properties or causing cells to produce a product. For example, Patent Document 1 describes a production method for a product including a step of culturing cells producing the product, which are contained in a cell suspension accommodated in a culture container; a separation treatment step of extracting the cell suspension from the culture container and separating the cell suspension using a separation membrane by a tangential filtration method; a step of returning a return solution to the culture container; a step of supplying a fresh culture medium into the culture container; and a step of recovering the product. In Patent Document 1, it is described that culturing is carried out such that, with respect to a viable cell concentration Nc, a pore diameter Dp of the separation membrane, a filtration area S of the separation membrane, and a volume Vf of a primary side flow channel of the separation membrane, a number density Nd of fine particles having a particle size of 8 Dp or more and 30 Dp or less other than viable cells in the cell suspension satisfies $Nc \leq Nd \leq S/(32 \times \pi \times Vf \times Dp^2)$.

Prior Art Documents

Patent Documents

Patent Document 1:WO2019/181234A

## SUMMARY OF THE INVENTION

### Object to be Solved by the invention

An alternating tangential flow filtration (ATF) method that is generally used in perfusion culture is less likely to cause membrane clogging (clogging of a hollow fiber side surface as evaluated in WO2019/181234A: hollow fiber side surface clogging) as compared with a tangential flow filtration (TFF) method due to a backwashing effect which is obtained by a liquid flowing back from a secondary side of a membrane to a primary side thereof. However, as developments for a purpose of productivity improvement progress, densification has progressed regarding the cell density, and hollow fiber side surface clogging is an issue even in the ATF method. A general measure for suppressing hollow fiber side surface clogging is to increase the membrane area. However, there is a problem in that commercially available membranes have a small area and devices are enlarged in size.

In examples of Patent Document 1, a permeation rate of an antibody is evaluated in a short period of 18 days in a small culture scale of 0.8 L. However, as a result of studies by the inventors of the present invention, it was found that there is a problem in that a cell viability decreases in a case where the culture scale is increased. It has been found that the decrease in the cell viability can be solved by increasing the aeration rate, but in a case where the aeration rate is increased, membrane clogging (primary clogging) occurs. An object to be achieved by the present invention is to provide a method that makes it possible to suppress membrane clogging (primary clogging: space clogging inside a hollow fiber) in a production method for a product by cell culture. The primary clogging is not clogging of pores on the hollow fiber side surface, which is evaluated by the permeation rate of the antibody of Patent Document 1, but is clogging of the space inside the hollow fiber, and increases a pressure loss of a hollow fiber membrane in a major axis direction.

### Means for solving the Object

As a result of diligent studies to achieve the above objects, the inventors of the present invention found that membrane clogging during perfusion culture can be suppressed by controlling a cumulative number of particles in a culture tank and an aeration rate in an appropriate region.

That is, according to the present invention, the following inventions are provided.

<1> A production method for a product, comprising:

culturing cells in a culture tank,

in which a cell density at a time of production of the product is $80 \times 10^6$ cells/mL or more and $300 \times 10^6$ cells/mL or less,

the culturing is perfusion culture,

a culture scale is 5 L or more and 100,000 L or less,

a cumulative number X [particles/mm$^2$] of particles having a particle diameter of 1.47 to 6.00 $\mu$m on Day 10 at the time of the production of the product per surface area of a membrane for separating cells and a product in a culture solution satisfies $0 < X \leq 1.0 \times 10^8$, and

an aeration rate V [vvm] satisfies $0.09 \leq V \leq 1.0$.

Where the cumulative number X is a total number of particles detected in a range of 1.47 to 6.00 $\mu$m in a case where a particle size distribution in 1.46 to 42 $\mu$m is measured for the culture solution.

<2> The production method for a product according to <1>, in which the cumulative number X [particles/mm$^2$] on Day 10 satisfies $0 < X \leq 9.7 \times 10^6$.

<3> The production method for a product according to <1>, in which the cumulative number X [particles/mm$^2$] on Day 10 satisfies $0 < X \leq 1.3 \times 10^6$.

<4> The production method for a product according to any one of <1> to <3>, in which a maximum cumulative number X [particles/mm$^2$] in a culture period after Day 10 satisfies $0 < X \leq 1.0 \times 10^8$.

<5> The production method for a product according to any one of <1> to <4>, in which a maximum cumulative number X [particles/mm$^2$] in a culture period after Day 10 satisfies $0 < X \leq 1.5 \times 10^6$.

<6> The production method for a product according to any one of <1> to <5>, in which a minimum cumulative number X [particles/mm$^2$] in a culture period after Day 10 satisfies $0 < X \leq 1.0 \times 10^8$.

<7> The production method for a product according to any one of <1> to <6>, in which a minimum cumulative number X [particles/mm$^2$] in a culture period after Day 10 satisfies $0 < X \leq 0.9 \times 10^6$.

<8> The production method for a product according to any one of <1> to <7>, in which a relationship between the cumulative number X [particles/mm$^2$] and the aeration rate V [vvm] satisfies $-10^4 \leq V - 10^5 \times X^{-0.9} \leq 0.1$.

<9> The production method for a product according to any one of <1> to <8>, in which a maximum shear rate D [1/s] applied to cells in a tube assembly from the culture tank to the membrane and in the membrane satisfies $0 < D \leq 65,000$.

<10> The production method for a product according to <9>, in which the maximum shear rate D [1/s] satisfies $0 < D \leq 39,000$.

<11> The production method for a product according to <9>, in which the maximum shear rate D [1/s] satisfies $0 < D \leq 3,000$.

<12> The production method for a product according to any one of <1> to <11>, in which a ratio [m$^2$/L] of a membrane area [m$^2$] per liquid amount [L] of the culture solution is 0.015 or more and 1.0 or less.

<13> The production method for a product according to any one of <1> to <12>, in which a time frequency T [s/day] at which a maximum shear rate is applied to cells in a tube assembly from the culture tank to the membrane and in the membrane satisfies $0 < T \leq 10,000$.

<14> The production method for a product according to any one of <1> to <13>, in which a cumulative time frequency T$_{total}$ [s/day] at which a shear rate of 100 [1/s] or more and 100,000 [1/s] or less is applied to cells in a tube assembly from the culture tank to the membrane and in the membrane satisfies $0 < T_{total} \leq 100,000$.

<15> The production method for a product according to any one of <1> to <14>, in which a relationship between a minimum inner diameter d [mm] of a tube and a joint of a tube assembly from the culture tank to the membrane and an amount C [L] of the culture solution satisfies $0.0001 \leq d/C \leq 100$.

<16> The production method for a product according to any one of <1> to <15>, in which a relationship between a length L [mm] of a flow channel, which is a minimum inner diameter d [mm], and an amount C [L] of the culture solution in a tube and a joint of a tube assembly from the culture tank to the membrane satisfies $0.001 \leq L/C \leq 1,000$.

<17> The production method for a product according to any one of <1> to <16>, in which a concentration [U/L] of a lactate dehydrogenase in the culture solution at the time of the production of the product is more than 0 and 100,000 or less.

<18> The production method for a product according to any one of <1> to <17>, in which a period of culturing the cells is 10 days or more and 100 days or less.

<19> The production method for a product according to any one of <1> to <18>, in which a period of culturing the cells is 15 days or more and 90 days or less.

<20> The production method for a product according to any one of <1> to <19>, in which a sparger pore diameter aerated in the culture solution is 1 to 100 $\mu$m.

<21> The production method for a product according to any one of <1> to <20>, in which a flux Y [LMH] during filtration satisfies $0 < Y \leq 10$.

<22> The production method for a product according to any one of <1> to <21>, in which the cell is an animal cell.

<23> The production method for a product according to any one of <1> to <22>, in which the cell is a CHO cell.

<24> The production method for a product according to any one of <1> to <23>, in which the product is an antibody.

<25> The production method for a product according to any one of <1> to <24>, in which a viscosity $\mu$ [mPa·s] of the culture solution at 37°C at the time of the production of the product satisfies $0.5 \leq \mu \leq 10$.

<26> The production method for a product according to any one of <1> to <25>, in which in the perfusion culture, after a cell density reaches a target cell density, which is $80 \times 10^6$ cells/mL or more, the culture solution containing the cells is extracted to maintain the cell density within ± 40% of the target cell density, and the product is recovered in a period in which the cell density is maintained within ± 40% of the target cell density.

<27> The production method for a product according to any one of <1> to <26>, further comprising: extracting the culture solution containing the cells at least once or more a day in the perfusion culture after a cell density reaches a target cell density, which is $80 \times 10^6$ cells/mL or more, and then adjusting the cell density to be within ± 10% of the target cell density.

<28> The production method for a product according to any one of <1> to <27>, in which in a period of at least 13 days or more in a period after a target cell density is reached at the time of the production of the product by the perfusion culture, A [mol/L/day], which is an alkali addition rate per day, is controlled in a range of $0 \leq A < 0.008$.

<29> The production method for a product according to any one of <1> to <28>, in which alkali addition is not carried out in an entire period of the perfusion culture.

<30> The production method for a product according to any one of <1> to <29>, in which an adding amount of an anti-foaming component per unit amount of the culture solution and per unit time is 0.70 mg/hour/L or less in a case where the anti-foaming component is added to the culture tank.

<31> The production method for a product according to <30>, in which the anti-foaming component is silicone-based.

<32> The production method for a product according to <30> or <31>, in which the anti-foaming component is dimethicone.

<33> The production method for a product according to any one of <1> to <32>, in which the membrane for separating the cells and the product in the culture solution is an alternating tangential flow: ATF method.

<34> A product that is produced by the production method for a product according to any one of <1> to <33>.

Effect of the Invention

According to the present invention, it is possible to suppress membrane clogging in perfusion culture.

## BRIEF DESCRIPTION OF THE DRAWINGS

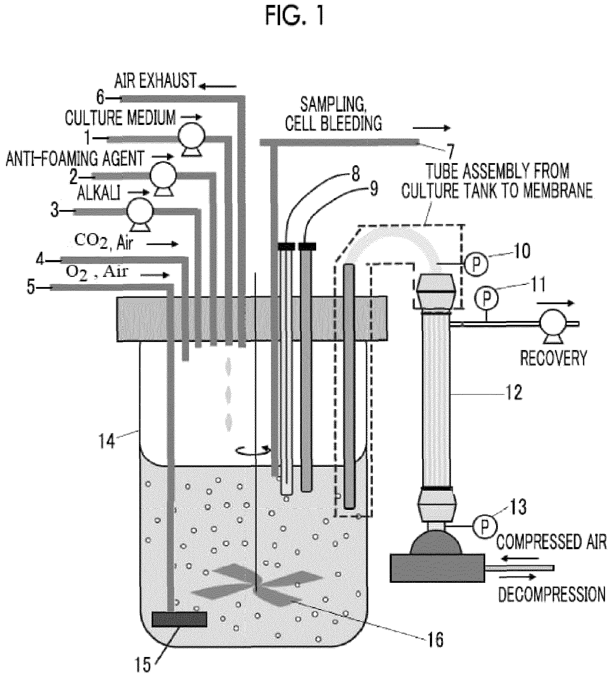

Fig. 1 shows a cell culture device. The entire device shown in Fig. 1 is a cell culture device.

## EMBODIMENTS FOR CARRYING OUT THE INVENTION

Hereinafter, the contents of the present invention will be described in detail. In the present specification, a numerical value range indicated using "to" means a range including numerical values described before and after "to" as a minimum value and a maximum value, respectively.

The present invention relates to a production method for a product, which includes culturing cells in a culture tank, in which a cell density at a time of production of the product is $80 \times 10^6$ cells/mL or more and $300 \times 10^6$ cells/mL or less, the culturing is perfusion culture, a culture scale is 5 L or more and 100,000 L or less, a cumulative number X [particles/mm$^2$] of particles having a particle diameter of 1.47 to 6.00 $\mu$m on Day 10 at the time of the production of the product per surface area of a membrane for separating cells and a product in a culture solution satisfies $0 < X \leq 1.0 \times 10^8$, and an aeration rate V [vvm] satisfies $0.09 \leq V \leq 1.0$.

According to the present invention, it is possible to improve productivity in perfusion culture using a membrane, particularly such as ATF/TFF.

In the present invention, the cumulative number X is a total number of particles detected in a range of 1.47 to 6.00 $\mu$m in a case where a particle size distribution in 1.46 to 42 $\mu$m is measured for the culture solution.

The particle size distribution in 1.46 to 42 $\mu$m can be measured with a precise particle size distribution measuring device Multisizer 4e (Beckman Coulter, Inc.).

From the viewpoint of suppressing the membrane clogging and lengthening the duration of culture, the cumulative number X [particles/mm$^2$] on Day 10 preferably satisfies $0 < X \leq 9.7 \times 10^6$ and more preferably satisfies $0 < X \leq 1.3 \times 10^6$.

The cumulative number X on Day 10 means the cumulative number X on Day 10, which is the 10th day from the start of culture, with the culture start date as Day 0.

From the viewpoint of suppressing the membrane clogging and lengthening the duration of culture, the maximum cumulative number X [particles/mm$^2$] in the culture period after Day 10 preferably satisfies $0 < X \leq 1.0 \times 10^8$ and more preferably satisfies $0 < X \leq 1.5 \times 10^6$.

The maximum cumulative number X in the culture period after Day 10 is the maximum value of the cumulative number X in a period after Day 10, which is the 10th day from the start of the culture, with the culture start date as Day 0.

From the viewpoint of suppressing the membrane clogging and lengthening the duration of culture, the minimum cumulative number X [particles/mm$^2$] in the culture period after Day 10 preferably satisfies $0 < X \leq 1.0 \times 10^8$, more preferably satisfies $0 < X \leq 1.0 \times 10^7$, still more preferably satisfies $0 < X \leq 6.0 \times 10^6$, and particularly preferably satisfies $0 < X \leq 0.9 \times 10^6$.

The minimum cumulative number X in the culture period after Day 10 is the minimum value of the cumulative number X in a period after Day 10, which is the 10th day from the start of culture, with the culture start date as Day 0.

In the present invention, the aeration rate V [vvm] satisfies $0.09 \leq V \leq 1.0$, and from the viewpoint of suppressing damage of breakage of bubbles to cells and reducing the number of fine particles, the aeration rate V preferably satisfies $0.09 \leq V \leq 0.5$ and more preferably satisfies $0.09 \leq V \leq 0.2$.

In the present invention, the maximum aeration rate V [vvm] satisfies $0.09 \leq V \leq 1.0$, preferably satisfies $0.09 \leq V \leq 0.5$, and more preferably satisfies $0.09 \leq V \leq 0.2$.

The aeration rate can be measured using an oxygen mass flow meter (model number: 8500MC-S1-1-2, gas species: O$_2$) and an air mass flow meter (model number: 8500MC-S1-1-2, gas species: Air), which are manufactured by KOFLOC.

The relationship between the cumulative number X [particles/mm$^2$] and the aeration rate V [vvm] preferably satisfies $-10^4 \leq V - 10^5 \times X^{-0.9} \leq 0.1$, more preferably satisfies $-10^2 \leq V - 10^5 \times X^{-0.9} \leq 0.1$, still more preferably satisfies $-10 \leq V - 10^5 \times X^{-0.9} \leq 0.1$, and particularly preferably satisfies $-4 \leq V - 10^5 \times X^{-0.9} \leq -0.01$.

The measuring method for the cumulative number X and the aeration rate V is as described above.

From the viewpoint of suppressing shear damage to cells and reducing the number of fine particles, the maximum shear rate D [1/s] applied to cells in the tube assembly from the culture tank to the membrane and in the membrane preferably satisfies $0 < D \leq 65,000$, more preferably satisfies $0 < D \leq 39,000$, and still more preferably satisfies $0 < D \leq 3,000$. The maximum shear rate D [1/s] may satisfy $2,000 < D \leq 65,000$ or $2,000 < D \leq 39,000$.

The shear rate D can be calculated according to the expression described in "(4) Shear rate (D) in tube assembly from culture tank to membrane and in membrane" in <Evaluation method> of examples described later.

The ratio [m$^2$/L] of the membrane area [m$^2$] per liquid amount [L] of the culture solution is preferably 0.015 or more and 1.0 or less, and more preferably 0.033 or more and 1.0 or less.

The liquid amount [L] of the culture solution can be measured or defined by a conventional method. The membrane area [m$^2$] can be measured by a conventional method, or a membrane having a predetermined defined membrane area can be used.

The time frequency T [s/day] at which the maximum shear rate is applied to cells in the tube assembly from the culture tank to the membrane and in the membrane preferably satisfies $0 < T \leq 10,000$, more preferably satisfies $0 < T \leq 3,500$, still more preferably satisfies $0.1 < T \leq 3,500$, and particularly preferably satisfies $0.1 < T \leq 1,000$.

The time frequency T can be calculated according to the expression described in "(5)Time frequency (T) in tube assembly from culture tank to membrane and in membrane" in <Evaluation method> of examples described later.

In the tube assembly from the culture tank to the membrane and in the membrane, the cumulative time frequency $T_{total}$ [s/day] at which a shear rate of 100 [1/s] or more and 100,000 [1/s] or less is applied to the cells preferably satisfies $0 < T_{total} \leq 100,000$, more preferably satisfies $0 < T_{total} \leq 10,000$, still more preferably satisfies $10 \leq T_{total} \leq 10,000$, and particularly preferably satisfies $100 \leq T_{total} \leq 10,000$.

The cumulative time frequency $T_{total}$ is the sum of the time frequencies T at each shear rate, the time frequency T can be calculated according to the expression described in "(5) Time frequency (T) in tube assembly from culture tank to membrane and in membrane" in <Evaluation method> of examples described later.

A relationship between a minimum inner diameter d [mm] of a tube and a joint of the tube assembly from the culture tank to the membrane and an amount C [L] of the culture solution preferably satisfies $0.0001 \leq d/C \leq 100$, more preferably satisfies $0.001 \leq d/C \leq 100$, and still more preferably satisfies $0.001 \leq d/C \leq 10$.

The minimum inner diameter d and the amount C of the culture solution can be measured or defined by a conventional method.

In the tube and the joint of the tube assembly from the culture tank to the membrane, a relationship between the length L [mm] of the flow channel, which is the minimum inner diameter d [mm], and the amount C [L] of the culture solution preferably satisfies $0.001 \leq L/C \leq 1,000$, more preferably satisfies $0.005 \leq L/C \leq 500$, still more preferably satisfies $0.01 \leq L/C \leq 100$, and particularly preferably satisfies $0.05 \leq L/C \leq 10$.

The minimum inner diameter d and the length L of the flow channel can be measured or defined by a conventional method.

In the present invention, the cell density at the time of the production of the product is $80 \times 10^6$ cells/mL or more and $300 \times 10^6$ cells/mL or less, preferably $90 \times 10^6$ cells/mL or more and $250 \times 10^6$ cells/mL or less, and more preferably 100

x $10^6$ cells/mL or more and 150 x $10^6$ cells/mL or less. M may be used to denote $10^6$.

The cell density can be measured by extracting the culture solution in the culture tank and subjecting the culture solution to measurement using a Cell Viability Analyzer Vi-cell XR manufactured by Beckman Coulter, Inc.

Preferably, in the present invention, in the perfusion culture described later, it is possible to extract a culture solution containing cells after a cell density reaches a target cell density, which is $80 \times 10^6$ cells/mL or more, thereby maintaining the cell density within ± 40% of the target cell density, and to recover the product in a period in which the cell density is maintained within ± 40% of the target cell density.

Preferably, it is possible to extract a culture solution containing cells at least once or more a day in the perfusion culture after a cell density reaches a target cell density, which is $80 \times 10^6$ cells/mL or more, and then adjust the cell density to be within ± 10% of the target cell density.

In the present invention, the mode of the method of culturing cells is perfusion culture. The perfusion culture is a culture method in which a fresh culture medium is supplied into a cell culture solution and a part of the culture medium in which cells are cultured is removed. In a case where this perfusion culture is carried out, it is possible to remove, from a culture tank, metabolic decomposition products discharged from cells. In the perfusion culture, it is possible to recover a liquid obtained by continuously separating cells in a culture solution while continuously supplying a culture medium to a culture tank.

In general, the perfusion culture makes it possible to achieve a high viable cell density. A typical perfusion culture begins with a batch culture that continues for 1 or 2 days, thereafter a fresh supply culture medium is added to the culture continuously, stepwise, and/or intermittently, and the used culture medium is removed at the same time. In the perfusion culture, it is also possible to separate cells by using methods such as sedimentation, centrifugation, and filtration and remove the used culture medium while maintaining the viable cell density. The advantage of the perfusion culture is that the culture in which a target protein is produced is maintained for a long period as compared with the batch culture method or the fed-batch culture.

Perfusion may be any form of being continuous, stepwise, intermittent, or a combination thereof. A continuous form is preferable. Animal cells are retained in the culture and the used culture medium that is removed may substantially not include cells or may have much fewer cells than the culture. A product that is expressed by cell culture can be retained in the culture or recovered by the selection of the membrane pore diameter.

The continuous separation method for a cell culture solution in the perfusion culture is preferably a method performing continuous separation using a membrane, and is more preferably membrane filtration. The membrane for separating the cells and the product in the culture solution is more preferably alternating tangential flow filtration (ATF method).

Y [L/m$^2$/hour], which is a flux during filtration, preferably satisfies $0 < Y \leq 10$, more preferably satisfies $0 < Y \leq 5$, and still more preferably satisfies $0 < Y \leq 2$.

The flux during filtration is an amount of a culture solution that permeates through a membrane per unit time and per unit filtration area, and it is defined by the following expression.

$$\text{Flux during filtration [L/m}^2\text{/hour]} = \frac{\text{Flow rate when passing through filtration membrane [L/hour]}}{\text{Area of filtration membrane [m}^2\text{]}}$$

The flow rate of the liquid passing through the filtration membrane can be measured by recording the weight of the liquid that has passed through the filtration membrane within a certain time. In a case where a gravimeter is not used, the flow rate can also be measured by a flowmeter.

The area of filtration membrane can be measured by a conventional method, or a filtration membrane having a predetermined area can be used.

The perfusion ratio is not particularly limited; however, it is generally 0.3 vvd to 5.0 vvd, preferably 0.5 vvd to 2.0 vvd, and more preferably 0.5 vvd to 1.4 vvd. vvd means an amount required for exchanging a cell culture solution per the volume of the cell culture solution for one day with a fresh culture medium, that is, it is "volume of supply culture medium/volume of culture solution/Day".

A method for extracting a product from a culture solution can be carried out by draining the product from the secondary side of the filtration membrane with a pump; however, another available liquid feeding means may be used. The extracted culture solution is subjected to treatments such as product recovery and removal of the dead cells. In addition, the extracted culture solution may be partially discarded or returned to the culture container after treatments such as product recovery and removal of the dead cells. In a case where a loss of the culture solution occurs due to the above treatments, the loss can be compensated, for example, by supplying a fresh culture medium to the culture container.

In the perfusion culture, it is possible to extract a culture solution containing cells at least once or more a day after a cell density reaches a target cell density, which is $80 \times 10^6$ cells/mL or more, and then adjust the cell density to be within ± 10% of the target cell density.

Extracting a part of a culture solution together with cells so that the viable cell density during the culturing does not become excessive, thereby reducing the viable cell density is referred to as cell bleeding, and the amount of the culture solution can be maintained by adding the same amount of a fresh culture medium as the amount of the extracted culture solution. The term "once or more a day" includes a case of continuously and automatically carrying out cell bleeding.

The period of culturing the cells is not particularly limited; however, it is generally 1 day or more and 1,000 days or less, preferably 7 days or more and 1,000 days or less, more preferably 10 days or more and 500 days or less, still more preferably 10 days or more and 100 days or less, even still more preferably 15 days or more and 90 days or less, and particularly preferably 20 days or more and 60 days or less.

The period of culture for the production of the product in which the cell density is $80 \times 10^6$ cells/mL or more is preferably 5 days or more and 990 days or less and may be 5 days or more and 450 days or less, and it is more preferably 10 days or more and 450 days or less, still more preferably 15 days or more and 190 days or less, and even still more preferably 20 days or more and 90 days or less.

In the present invention, an alkali may be added during the perfusion culture. Meanwhile, in the entire period of the perfusion culture, it is possible to carry out culture without alkali addition.

In the present invention, in a period of at least 13 days or more in a period after a target cell density is reached at the time of the production of the product by the perfusion culture, A [mol/L/day], which is an alkali addition rate per day, may be controlled in a range of $0 \leq A < 0.008$.

The alkali is not particularly limited; however, it is preferably $Na_2CO_3$, NaOH, or $NaHCO_3$, more preferably $NaHCO_3$ and/or $Na_2CO_3$, and particularly preferably $NaHCO_3$. The alkali can be added as an aqueous solution (for example, an $Na_2CO_3$ aqueous solution, an NaOH aqueous solution, or an $NaHCO_3$ aqueous solution).

The pH of the aqueous alkali solution to be added satisfies $7 < pH < 13$, preferably satisfies $7.5 < pH < 12$, more preferably satisfies $7.5 < pH < 10$, and still more preferably satisfies $8 < pH < 10$.

The pH of the aqueous alkali solution is measured at a time when the alkali has dissolved in water. The pH can be measured using, in general, a commercially available pH sensor. Examples thereof include a pH meter (Seven Excellence, Seven Direct, Five Easy) manufactured by METTLER TOLEDO.

The pH of the culture medium to be added in the perfusion culture is preferably 7.0 to 8.0, more preferably 7.0 to 7.8, and still more preferably 7.0 to 7.6.

The pH of the culture medium can be measured using, in general, a commercially available pH sensor after storing the culture medium for 1 day in incubation at 5% $CO_2$ and 37°C. Examples thereof include a pH meter (Seven Excellence, Seven Direct, Five Easy) manufactured by METTLER TOLEDO.

The average pH of the culture solution during the culturing is preferably 6.7 to 7.2 and more preferably 6.8 to 7.0.

The minimum pH of the culture solution during the culturing is preferably 6.6 or more, more preferably 6.7 or more, and still more preferably 6.8 or more.

Preferably, it is possible to control a pH of a culture solution during the culturing by automatically adding an aqueous alkali solution while subjecting a pH in the culture solution to an in-line measurement.

Preferably, an anti-foaming agent can be added in the perfusion culture. The anti-foaming component of the anti-foaming agent is preferably silicone-based, and it is particularly preferably dimethicone. The anti-foaming component of the anti-foaming agent is preferably an anti-foaming component containing polydimethylsiloxane, and it is more preferably an anti-foaming component in which fine powder silica is contained in polydimethylsiloxane. As the anti-foaming agent, it is possible to use, for example, HyClone ADCF Antifoam Agent manufactured by Cytiva.

In the perfusion culture, the anti-foaming agent may be added from the start of the culture, or may be added starting at a predetermined time after the start of the culture (for example, from the 1st day to the 10th day after the start of the culture, preferably from the 2nd day to the 9th day after the start of the culture, more preferably from the 3rd day to the 8th day after the start of the culture, and particularly preferably from the 5th day to the 7th day after the start of the culture).

The addition rate of the anti-foaming agent is not particularly limited; however, the adding amount of the anti-foaming component per unit amount of the culture solution and per unit time in a case where the anti-foaming component is added to the culture tank is preferably 0.70 mg/hour/L or less, more preferably 0.07 mg/hour/L or more and 0.70 mg/hour/L or less, and still more preferably 0.2 mg/hour/L or more and 0.7 mg/hour/L or less. In addition, the addition rate of the dimethicone in a case of being added is preferably 2 mg/day/L to 30 mg/day/L, more preferably 5 mg/day/L to 20 mg/day/L, and still more preferably 8 mg/day/L to 15 mg/day/L.

Fig. 1 shows one example of a cell culture device that can be used in the cell culture in the present invention. In Fig. 1, a culture container 14 is a container that accommodates a culture solution containing cells. Cells are cultured in the culture solution in the inside of the culture container 14.

The culture medium is supplied to the culture container from a culture medium supply pipe 1.

An anti-foaming agent for suppressing foaming is supplied from an anti-foaming agent supply pipe 2 to the culture container.

An alkali is supplied from an alkali supply pipe 3 to the culture container. In a case where the alkali is not added, the alkali supply pipe 3 may not be provided.

Carbon dioxide ($CO_2$) and air are introduced from an air supply pipe 4 into an upper part of the culture solution in the side of the culture container.

Oxygen ($O_2$) and/or air is sent from a sparger air supply pipe 5, and the oxygen and/or air is introduced into the culture solution through a sparger 15 having a pore diameter of 20 $\mu$m. The dissolved oxygen concentration inside the culture solution can be adjusted by the sparger 15. The sparger pore diameter (pore diameter of gas release unit) is preferably 1 to 300 $\mu$m, more preferably 1 to 100 $\mu$m, still more preferably 5 to 50 $\mu$m, particularly preferably 10 to 30 $\mu$m, and 20 $\mu$m as an example. As the sparger, preferably, a sparger that releases a gas containing 30% by volume or more of oxygen can be used.

An exhaust pipe 6 is a pipe for exhausting air, and an exhaust filter (not shown in the drawing) may be connected to a terminal thereof.

The sampling tube 7 is a pipe for collecting (sampling) the culture solution or extracting (cell bleeding) the culture solution. In a case where the cell is automatically and continuously subjected to cell bleeding, a pipe may be separately installed (not shown in the drawing).

A pH sensor 8 is mounted to come into contact with the culture solution.

A dissolved oxygen sensor 9 is mounted to come into contact with the culture solution.

Pressure sensors 10, 11, and 13 may be provided in the cell culture device.

A hollow fiber membrane 12 is installed in the cell culture device.

A region surrounded by a dotted line indicates the tube assembly from the culture container (culture tank) to the membrane.

A stirring member having a stirring blade 16 may be provided inside the culture container 14. In a case where the stirring blade 16 is rotated, the culture solution inside the culture container 14 is stirred, and the homogeneity of the culture solution is maintained. In a case where the culture solution is stirred by the stirring blade 16, the bubbles released by the sparger are also stirred. The position of the stirring member having a stirring blade, the size of the stirring blade, and the like are not particularly limited and may be designed depending on the cell kind to be used, the amount of the culture solution, the amount of oxygen to be supplied, or the position, number, size, or the like of the sparger. Further, in order to quickly stir bubbles coming out of the sparger and suppress coalescence of the bubbles, it is preferable to dispose the stirring blade 16 at a position close to the sparger.

In the present invention, a cell suspension extracted from the culture container may be allowed to pass through a separation membrane to separate the cell suspension into a cell-containing liquid and a permeated solution. This operation can be carried out using a cell culture device. In this operation, the cell suspension extracted from the culture container is separated into a cell-containing liquid having a cell concentration higher than that of the cell suspension and a permeated solution having a cell concentration lower than that of the cell suspension. The cell concentration can be measured by a cell viability analyzer, Vi-CELL XR, manufactured by Beckman Coulter Life Sciences.

The membrane separation treatment step described above is preferably tangential filtration, more preferably alternating tangential flow filtration or tangential flow filtration, and most preferably alternating tangential flow filtration. Examples of the filter that makes it possible to carry out alternating tangential flow filtration include SuATF10-S02PES and F2 RF02PES, which are manufactured by Repligen Corporation.

As the culture medium that is used for cell culture, a culture medium that is generally used for culturing animal cells can be used. For example, CD OptiCHO (manufactured by Thermo Fisher Scientific, Inc.), Dulbecco's modified Eagle medium (DMEM), Eagle minimum essential medium (MEM), RPMI-1640 medium, RPMI-1641 medium, F-12K medium, Ham's F12 medium, Iscove's modified Dulbecco's medium (IMDM), McCoy's 5A medium, Leibovitz's L-15 medium, and EX-CELL (trade mark) 300 series (JRH Biosciences), CHO-S-SFMII (Invitrogen), CHO-SF (Sigma-Aldrich Co. LLC), CD-CHO (Invitrogen), ISCHO-V (FUJIFILM Irvine Scientific), PF-ACF-CHO (Sigma-Aldrich Co. LLC), and the like can be used. Alternatively, a homemade culture medium may be used.

Serum such as fetal calf serum (FCS) may be added to the culture medium, or serum may not be added thereto. The culture medium may be supplemented with additional components such as an amino acid, salts, a sugar, a vitamin, a hormone, a growth factor, a buffer solution, an antibiotic, a lipid, a trace element, and a hydrolysate of a plant protein. A protein-free culture medium can also be used.

The culture temperature is generally 30°C to 40°C, preferably 32°C to 39°C, and more preferably 36°C to 38°C, and the culture temperature may be changed during the culturing.

The culture can be carried out in an atmosphere having a $CO_2$ concentration of 0% to 40% by volume, preferably 2 to 25% by volume, and more preferably 3 to 20% by volume.

The culture scale is preferably 5 L or more and 100,000 L or less, more preferably 50 L or more and 100,000 L or less, still more preferably 500 L or more and 100,000 L or less, and even still more preferably 1,000 L or more and 100,000 L or less. The upper limit of the culture scale is preferably 10,000 L and more preferably 5,000 L.

In the culture, the culture medium can be replaced, aerated, and stirred as necessary. In a case of carrying out stirring of the culture solution, the rotation speed of stirring is not particularly limited; however, the stirring power per unit volume is generally 10 to 300 kW/m$^3$, preferably 20 to 200 kW/m$^3$, and more preferably 30 to 100 kW/m$^3$.

The dissolved oxygen concentration in the culture solution can be appropriately set and is not particularly limited; however, it is generally 10% to 100% and preferably 30% to 90%.

In addition, a dissolved $CO_2$ concentration in a period in which the cell density is maintained at $80 \times 10^6$ cells/mL or more is preferably 60 to 180 mmHg, more preferably 80 to 160 mmHg, and still more preferably 100 to 140 mmHg.

The cell culture can be carried out using a cell culture device having the configuration described above in the present specification. The cell culture device may be any one of a fermenter tank type culture device, an air lift type culture device, a culture flask type culture device, a spinner flask type culture device, a microcarrier type culture device, a fluidized bed type culture device, a hollow fiber type culture device, a roller bottle type culture device, a filling tank type culture device, or the like. The culture container is preferably a single-use culture tank from the viewpoint of homogenizing the culture environment or the like.

A concentration of lactate dehydrogenase (LDH) in a culture solution at the time of the production of the product (maximum concentration of lactate dehydrogenase) [U/L] is preferably more than 0 and 100,000 or less, more preferably 10 or more and 30,000 or less, and still more preferably 100 or more and 5,000 or less.

The concentration of lactate dehydrogenase (LDH) in the culture solution can be measured by extracting the culture solution, separating cells and supernatant at 300 G/5 minutes, and using Cedex Bio manufactured by F. Hoffmann-La Roche, Ltd for the supernatant.

The viscosity $\mu$ [mPa·s] of the culture solution at 37°C at the time of the production of the product preferably satisfies $0.5 \leq \mu \leq 10$, more preferably satisfies $1.0 \leq \mu \leq 5$, and still more preferably satisfies $1.3 \leq \mu \leq 3$.

The viscosity of the culture solution can be measured by maintaining the culture solution extracted from the culture tank at 37°C and using a vibration type viscometer (model number: VM-10A) manufactured by SEKONIC CORPORATION.

The kind of the cell in the present invention is not particularly limited; however, examples thereof include eukaryotic cells such as an animal cell, a plant cell, and yeast, prokaryotic cells such as Bacillus subtilis, and Escherichia coli. The cell is preferably an animal cell (more preferably a mammalian cell) or an insect cell, and it is most preferably a mammalian cell. The cell may be a primary cell or a cell established as a cell line.

Examples of the cell include a Chinese hamster ovary (CHO) cell, a HEK cell (a cell derived from the human embryonic kidney), a BHK cell, a 293 cell, a C127 cell, a myeloma cell (such as an NS0 cell), a PerC6 cell, an SP2/0 cell, a hybridoma cell, a COS cell (a cell derived from the kidney of the African green monkey), a 3T3 cell, a HeLa cell, a Vero cell (a renal epithelial cell of the African green monkey), a MDCK cell (a cell derived from a canine kidney renal tubular epithelial cell), a PC12 cell, and a WI38 cell. The cell may be a stem cell such as an embryonic stem cell (ES cell) or an induced pluripotent stem cell (iPS cell). Among these, a CHO cell, a HEK cell, a BHK cell, or a hybridoma is preferable, where a CHO cell or a HEK cell is more preferable, and a CHO cell is most preferable. The CHO cell is widely used for the production of recombinant proteins such as a cytokine, a coagulation factor, and an antibody. It is preferable to use a CHO cell deficient in dihydrofolate reductase (DHFR), and as a DHFR-deficient CHO cell, it is possible to use, for example, CHO-DG44.

It is preferable that the cell viability is high; however, it is preferably 85% or more, more preferably 90% or more, particularly preferably 95% or more, and most preferably 99% or more.

These cells may be cells into which a foreign gene encoding a protein desired to be expressed (for example, an antibody) has been introduced. The cell is preferably a cell that produces an antibody. An expression vector can be used for introducing a foreign gene encoding a protein desired to be expressed, into a cell. An expression vector containing a DNA encoding a protein desired to be expressed, an expression control sequence (for example, an enhancer, a promoter, a terminator, or the like), and a selection marker gene as desired is introduced into a cell, whereby it is possible to prepare a cell into which a foreign gene encoding a protein desired to be expressed is introduced. The expression vector is not particularly limited and can be appropriately selected and used depending on the kind, use application, and the like of the cell.

As the promoter, it is possible to use any promoter of which the function can be exhibited in mammalian cells. Examples thereof include a promoter of the immediate early (IE) gene of the cytomegalovirus (CMV), an early promoter of SV40, a retrovirus promoter, a metallothionein promoter, a heat shock promoter, an SR$\alpha$ promoter, and a promoter and enhancer of the Moloney murine leukemia virus. In addition, an enhancer of the IE gene of human CMV may be used together with the promoter.

As the selection marker gene, it is possible to use, for example, a drug resistance gene (a neomycin resistance gene, a dihydrofolate reductase (DHFR) gene, a puromycin resistance gene, a blasticidin resistance gene, a hygromycin resistance gene, a cycloheximide resistance gene, or the like), or a fluorescence gene (a gene encoding a green fluorescent protein GFP or the like).

The method of introducing an expression vector into a cell is not particularly limited, and it is possible to use, for example, a calcium phosphate method, an electroporation method, a liposome method, a gene gun method, or a lipofection method.

The production method for a product according to the embodiment of the present invention includes culturing a cell using a cell culture device to produce a product from the cell.

According to the present invention, there is provided a product that is produced by the production method for a product according to the embodiment of the present invention.

In the present invention, the kind of the product is not particularly limited; however, it is preferably a protein and more preferably a recombinant protein. Examples of the product include a recombinant polypeptide chain, a recombinant secreted polypeptide chain, an antigen-binding protein, an antibody (for example, a human antibody, a humanized antibody, a chimeric antibody, a mouse antibody, or a bispecific antibody), an Fc fusion protein, a fragmented immunoglobulin, and a single-chain antibody (scFv). In addition, it may be, in addition to the above, an adenovirus, an adeno-associated virus, a lentivirus, or the like.

The product is preferably an antibody, and more preferably a human antibody, a humanized antibody, a chimeric antibody, or a mouse antibody. Examples of the fragmented immunoglobulin include Fab, F(ab')2, and Fv. The class of the antibody is also not particularly limited, and it may be any class of IgG such as IgG1, IgG2, IgG3, or IgG4, IgA, IgD, IgE, or IgM. However, IgG or IgM is preferable in a case of being used as a medicine.

The human antibody includes all antibodies having one or a plurality of variable and constant regions induced from human immunoglobulin sequences. In one embodiment, all variable and constant domains are induced from human immunoglobulin sequences (complete human antibodies).

In a case of being administered to a human subject, the humanized antibody has a sequence different from a sequence of an antibody induced from a non-human species by substitution, deletion, and/or addition of one or a plurality of amino acids so that there is a low possibility for the humanized antibody to induce an immune response and/or so that induction of a severe immune response is reduced as compared with the antibody of non-human species. In one example, specific amino acids in a framework and constant domains of heavy chains and/or light chains of an antibody of non-human species are mutated to produce a humanized antibody. In another example, a constant domain from a human antibody is fused to a variable domain of an antibody of a non-human species.

The chimeric antibody is an antibody in which variable regions and constant regions having origins different from each other are linked. For example, an antibody consisting of variable regions of heavy chains and light chains of a mouse antibody and consisting of constant regions of heavy chains and light chains of a human antibody is a mouse/human heterologous chimeric antibody. It is possible to prepare a recombinant vector expressing a chimeric antibody by linking a DNA encoding variable regions of a mouse antibody and a DNA encoding constant regions of a human antibody and then incorporating the linked DNA into an expression vector. It is possible to acquire a chimeric antibody produced during the culturing by culturing a recombinant cell transformed with the above vector and expressing the incorporated DNA.

The bispecific antibody is an antibody that recognizes two kinds of antigenic specificity, which are different from each other. Various forms of bispecific antibodies are present. As a method of preparing a bispecific antibody, it has been reported a method of preparing a bispecific antibody by binding two immunoglobulin molecules by using a crosslinking agent such as N-succinimidyl 3-(2-pyridyldithiol)propionate or S-acetylmercaptosuccinic acid anhydride, a method of preparing a bispecific antibody by binding Fab fragments of immunoglobulin molecules to each other. In addition, a bispecific antibody can be expressed by introducing a gene encoding the bispecific antibody into a cell.

The Fc fusion protein indicates a protein having an Fc region and includes an antibody.

The Fab is a monovalent fragment having VL, VH, CL, and CH1 domains.

The F(ab')2 is a divalent fragment having two Fab fragments bound by a disulfide crosslinking at a hinge region.

The Fv fragment has VL and VH domains of a single arm of an antibody.

The single-chain antibody (scFv) is an antibody in which VL and VH regions are joined through a linker (for example, a synthetic sequence of amino acid residues) to form a continuous protein chain, where the linker is long enough to allow the protein chain to fold for itself and form a monovalent antigen binding site.

The antibody is not particularly limited; however, examples thereof include an anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, and an anti-VLA4 antibody.

Regarding the product recovery, the culture solution may be simply recovered. For example, a liquid obtained by removing at least a part of the cells from the culture solution using a filter or a centrifuge may be recovered, and a known method is used without particular limitation. In a case of desiring to improve the purity of the product, change the solvent of the product, or change the form of the product to, for example, a powder form, the culture solution or the liquid can be subjected to further treatment.

In addition, a part of the culture solution can be recovered while being perfused, or a liquid which is obtained by removing at least a part of cells from the part of the culture solution while being perfused can be recovered by using a filter or a centrifuge.

The product can be purified by a purification treatment. The obtained product can be purified to high purity. For the separation and purification of the product, the separation and purification methods that are used for general proteins may be used. For example, it is possible to carry out separation and purification of a product by appropriately selecting and combining means such as chromatography such as affinity chromatography, a filter, ultrafiltration, salting out, dialysis, sodium dodecyl sulfate (SDS) polyacrylamide gel electrophoresis, isoelectric focusing electrophoresis, and the like, which are not limited thereto. The concentration of the product obtained as above can be measured according to absorbance measurement, enzyme-linked immunosorbent assay (ELISA), or the like. In addition, in a case where the product is an antibody, the titer of the antibody can also be measured with a commercially available analytical instrument such as Cedex Bio manufactured by F. Hoffmann-La Roche, Ltd.

Examples of the column that is used for affinity chromatography include a protein A column and a protein G column. Examples of the chromatography other than affinity chromatography include ion exchange chromatography, hydrophobic chromatography, gel filtration, reverse phase chromatography, and adsorption chromatography. The chromatography can be carried out using liquid phase chromatography such as high performance liquid chromatography (HPLC) or fast protein liquid chromatography (FPLC).

It is noted that it is also possible to modify the product or partially remove a peptide thereof by allowing an appropriate polypeptide modifying enzyme to act on the product before or after purification. As the polypeptide modifying enzyme, for example, trypsin, chymotrypsin, lysyl endopeptidase, protein kinase, or glucosidase is used.

The product that is produced according to the present invention can be used, for example, for a biopharmaceutical drug, or regenerative medicine.

The present invention will be more specifically described using the following examples; however, it is not limited by the examples.

Examples

<Establishment of antibody-producing cell>

A vector containing a nucleic acid sequence encoding each of IgG1 and IgG4 was constructed, and the constructed vector was introduced into a CHO-DG44 cell to prepare a CHO-DG44 cell expressing IgG1 (an IgG1 cell) and a CHO-DG44 cell expressing IgG4 (an IgG4 cell). The construction of the vector and the introduction thereof into the cell were carried out according to Example 2 of JP2016-517691A. As described above, CHO cells producing monoclonal antibodies were prepared and used in the following experiment.

<Culture container>

Examples 1 to 4:

Culture container: A plastic single-use culture container having a diameter of 349 mm and a tank height of 685 mm, where the stirring blade is a propeller blade having a diameter of 116 mm

Filtration: A F4 RF02PES-V2 membrane was used in a system of ATF4, manufactured by Repligen Corporation. Regarding the F4 RF02PES-V2 membrane, the pore diameter is 0.2 $\mu$m, the hollow fiber diameter is 1 mm, and the filtration area is 0.77 m$^2$.

Example 5:

Culture container: A glass culture container having a diameter of 225 mm and a tank height of 400 mm, where the stirring blade is a paddle blade having a diameter of 150 mm

Filtration: A F2 RF02PES membrane was used in a system of ATF2, manufactured by Repligen Corporation. Regarding the F2 RF02PES membrane, the pore diameter is 0.2 $\mu$m, the hollow fiber diameter is 1 mm, and the filtration area is 0.13 m$^2$.

<Cell culture>

CHO cells are seeded at $0.5 \times 10^6$ cells/ml. As the culture medium, CD OptiCHO (manufactured by Thermo Fisher Scientific, Inc.) was used.

$O_2$ from the bottom surface was automatically controlled and supplied from a sparger installed in the culture tank so that the oxygen concentration in the culture solution was 90%. An aqueous alkali solution was not added during the culture period.

After culturing for 2 days after seeding, while continuously supplying a culture medium at a perfusion ratio of 0.8 vvd, ATF manufactured by Repligen Corporation was operated, the cell culture solution was continuously filtered, and the recovery liquid was recovered.

Further, on the 5th day, the perfusion ratio was changed to 1.2 vvd.

Further, on the 6th day, the addition rate of dimethicone in the anti-foaming agent was changed to 12.0 mg/day/L.

In a case where the cell density reached $120 \times 10^6$ cells/ml, the cell bleeding was carried out while a part of the culture solution was drained so that the cell density was maintained at $120 \times 10^6$ cells/ml.

The culture was continued until the membrane clogging occurred and the ATF could not operate.

The culture conditions are described in the following table.

<Evaluation method>

(1) Measurement of cell density and viability

The culture solution in the culture tank was extracted and subjected to measurement using Cell Viability Analyzer Vi-cell XR manufactured by Beckman Coulter, Inc. It is noted that for the Vi-cell software, Vi-cell XR2.04 was used, and the parameters at the time of measurement were set as follows.

Min diameter: 6 $\mu$m

Max diameter: 50 $\mu$m

Dilution: In a case where the cell concentration was $10 \times 10^6$ cells/mL or less, the sample was not diluted, and the Dilution was set to 1. In a case where the cell concentration was higher than $10 \times 10^6$ cells/mL, the sample was diluted 10 times, and the Dilution was set to 10.

Cell brightness: 75%,

Cell sharpness: 100

Viable cell spot brightness: 75%

Viable cell spot area: 5%

Minimum circularity: 0

Decluster degree: Medium

(2) Measurement of pH and dissolved oxygen concentration

The culture solution in the culture tank was extracted and subjected to measurement using RAPIDLab 348EX, manufactured by SIEMENS AG.

(3) Filtration flux and reciprocating flow rate in ATF

The filtration flux was measured by recording the weight of the liquid that passed through the filtration membrane within a certain time. The reciprocating flow rate was controlled by the controller with a set value input to the controller.

(4) Shear rate (D) in tube assembly from culture tank to membrane and in membrane

$$\mathbf{Re} = \frac{\rho \times u \times d}{\mu}$$ **Re** :Reynolds number [-]

① In case of laminar flow (Re < 3000)

$$D = \frac{8 \times u}{d}$$

**D :** Shear rate [1/s]
**u :** Averageflow rate in flow channel [m/s]
**d :** Flow channel inner diameter [m]

② In case of turbulent flow (Re ≥ 3000)

$$D = \frac{\tau}{\mu}$$

$$\tau = 0.022 \times \rho \times u_{max}^2 \times \frac{v}{u_{max} \times d}$$

$$u_{max} = \frac{u}{0.8}$$

$\tau$ : Shear stress [**Pa**]

$\mu$ : Viscosity [Pa·s]
$\rho$ : Density [kg/m³]
$u_{max}$ :Maximum flow rate in flow channel [m/s]
v: Kinematic viscosity [m²/s]

(5) Time frequency (T) in tube assembly from culture tank to membrane and in membrane

$$T = N \times t$$

$$N = \frac{U}{V}$$

$$t = \frac{v}{U'}$$

**T :** Time at which shear rate D is received per day **[s/day]**
**N** : Number of times shear rate D is received per day [times/day]
**U** : Flow rate through flow channel [m³/day]
**V** : Amount of culture solution [m³]
**t** :Time at which shear rate D is received per day [s/times]
**U'** : Flow rate through flow channel [m³/s]
**v** :Flow channel volume in which shear rate D is applied [m³]

(6) Cumulative shear frequency

The cumulative time frequency $T_{total}$ was calculated by calculating the sum of the time frequencies T at each shear rate.
That is, the cumulative time frequency $T_{total}$ represents the total time for which one cell per day is subjected to the shear of 100 to 100,000 [1/s].

(7) Measurement of fine particle density

The culture solution in the culture tank was extracted and diluted 10,000 times with ISOTON (Beckman Coulter, Inc.). The diluted culture solution was subjected to a measurement of a particle size distribution in 1.46 to 42 $\mu$m with a precise particle size distribution measuring device Multisizer 4e (Beckman Coulter, Inc.). The data is output in increments of about 0.0124 $\mu$m. The cumulative number of particles of 1.47 to 6.00 $\mu$m is the total number of particles detected in a range of 1.47 to 6.00 $\mu$m.

(8) Measurement of lactate dehydrogenase (LDH)

The culture solution was extracted, and the cells and the supernatant were separated from each other at 300 G/5 minutes. The supernatant was subjected to measurement with Cedex Bio manufactured by F. Hoffmann-La Roche, Ltd.

(9) Measurement of viscosity

The culture solution in the culture tank was extracted, the extracted culture solution was maintained at 37°C and the viscosity was measured using a vibration type viscometer (model number: VM-10A) manufactured by SEKONIC CORPORATION.

EP 4 596 704 A1

(10) Measurement of density ($\rho$) used for calculating Reynolds number Re

1 ml of the solution was quantified with a micropipette (model number: 4920 000.083) manufactured by Eppendorf, and the weight thereof was measured with a balance (ML6002T/00) manufactured by METTLER TOLEDO. The above-described weight was multiplied by 1,000 to obtain a density [$kg/m^3$].

(11) Aeration rate

Measurement was performed using an oxygen mass flow meter (model number: 8500MC-S1-1-2, gas species: $O_2$) and an air mass flow meter (model number: 8500MC-S1-1-2, gas species: Air), which are manufactured by KOFLOC.

(12) Pressure

The pressure on the ATF primary side was measured with ACPM-05TC-01N manufactured by Repligen Corporation. The pressure on the ATF secondary side was measured with ACPM-799-01N manufactured by Repligen Corporation. The maximum differential pressure on the primary side up to day 30 is shown in the following table.

(13) Evaluation of membrane clogging

The evaluation standards for membrane clogging are shown below.

A: In a case where the number of days taken from the start of the culture to the clogging is 30 days or more.
B: In a case where the number of days taken from the start of the culture to the clogging is 20 days or more and 29 days or less.
C: In a case where the number of days taken from the start of the culture to the clogging is 10 days or more and 19 days or less.

<Culture conditions and results>

Culture conditions and results for Examples 1 to 5 are shown in the following table.

[Table 1]

| | | | Example 1 | Example 2 | Example 3 | Example 4 | Example 5 |
|---|---|---|---|---|---|---|---|
| Culture conditions | | Scale | L | 45 | 45 | 45 | 45 | 7.1 |
| | | Cell density | $10^6$ cells/ml | 120 | 120 | 120 | 120 | 120 |
| | ATF | Hollow fiber membrane model number | | F4 RF02PES-V2 | F4 RF02PES-V2 | F4 RF02PES-V2 | F4 RF02PES-V2 | F2 RF02PES |
| | | Number used at same time | | 2 | 1 | 2 | 2 | 2 |
| | | Inner diameter | Mm | 1 | 1 | 1 | 1 | 1 |
| | | Membrane area | $m^2$ | 1.54 | 0.77 | 1.54 | 1.54 | 0.26 |
| | | Membrane area per liquid amount | $m^2$/L | 0.034 | 0.017 | 0.034 | 0.034 | 0.037 |
| | | Filtration flux | LMH | 1.5 | 2.9 | 1.5 | 1.5 | 1.4 |
| | | Reciprocating flow rate | LPM | 6 | 6 | 6 | 8 | 0.7 |
| | Flow channel of minimum inner diameter from culture tank to filtration device | Minimum inner diameter | Mm | 9.5 | 9.5 | 5.5 | 5.5 | 5.5 |
| | | Inner diameter per liquid amount | mm/L | 0.21 | 0.21 | 0.12 | 0.12 | 0.77 |
| | | Length | Mm | 130 | 130 | 8 | 8 | 8 |
| | | Length per liquid amount | mm/L | 2.9 | 2.9 | 0.18 | 0.18 | 1.1 |
| | Microsparger aeration | DO (dissolved oxygen concentration) | % | 90 | 90 | 90 | 90 | 90 |
| | | Sparger diameter | μm | 25 | 25 | 25 | 25 | 20 |
| | | Maximum aeration rate | vvm | 0.16 | 0.16 | 0.16 | 0.16 | 0.16 |

[Table 2]

| | | | Example 1 | Example 2 | Example 3 | Example 4 | Example 5 |
|---|---|---|---|---|---|---|---|
| Results | Maximum shear | Location of maximum shear occurrence | Connection flow channel | Connection flow channel | Connection flow channel | Connection flow channel | Hollow fiber membrane |
| | | Maximum shear rate, 1/s | 2370 | 2370 | 38971 | 64473 | 1580 |
| | | Time frequency, s/day | 32 | 16 | 0.73 | 0.73 | 801 |
| | Cumulative time frequency | s/day | 970 | 485 | 970 | 970 | 2012 |
| | Viscosity | Maximum (after day 10), mPa•s | 1.54 | 1.78 | 1.62 | 1.58 | 1.83 |
| | | Minimum (after day 10), mPa•s | 2.79 | 2.61 | 2.82 | 2.85 | 2.49 |
| | Number of fine particles per membrane area | Day 10, $10^6$ particles/mm$^2$ | 0.28 | 0.46 | 4.8 | 8.8 | 0.96 |
| | | Maximum (after day 10), $10^6$ particles/mm$^2$ | 0.64 | 1.1 | 5 | 10 | 1.2 |
| | | Minimum (after day 10), $10^6$ particles/mm$^2$ | 0.21 | 0.33 | 2.5 | 4.2 | 0.89 |
| | Maximum LDH | U/L | 794 | 1680 | 1835 | 4162 | 1760 |
| | Duration of culture | Number of days | 33 | 27 | 28 | 16 | 30 |
| | Evaluation | - | A | B | B | C | A |

<Explanation of result>

In Examples 1 to 5, the duration of culture was 16 days or more. From the above results, it has been verified that the culture can be continued in a state where the clogging of the membrane during perfusion culture is suppressed by controlling the cumulative number of particles (shear rate) in the culture tank and the aeration rate in an appropriate region.

Explanation of References

1: culture medium supply pipe
2: anti-foaming agent supply pipe
3: alkali supply pipe
4: air supply pipe
5: sparger air supply pipe
6: exhaust pipe
7: sampling tube
8: pH sensor
9: dissolved oxygen sensor
10: pressure sensor
11: pressure sensor
12: hollow fiber membrane
13: pressure sensor
14: culture container
15: sparger
16: stirring blade

## Claims

1. A production method for a product, comprising:

   culturing cells in a culture tank,
   wherein a cell density at a time of production of the product is $80 \times 10^6$ cells/mL or more and $300 \times 10^6$ cells/mL or less,
   the culturing is perfusion culture,
   a culture scale is 5 L or more and 100,000 L or less,
   a cumulative number X [particles/mm$^2$] of particles having a particle diameter of 1.47 to 6.00 $\mu$m on Day 10 at the time of the production of the product per surface area of a membrane for separating cells and a product in a culture solution satisfies $0 < X \leq 1.0 \times 10^8$, and
   an aeration rate V [vvm] satisfies $0.09 \leq V \leq 1.0$,
   where the cumulative number X is a total number of particles detected in a range of 1.47 to 6.00 $\mu$m in a case where a particle size distribution in 1.46 to 42 $\mu$m is measured for the culture solution.

2. The production method for a product according to claim 1,
   wherein the cumulative number X [particles/mm$^2$] on Day 10 satisfies $0 < X \leq 9.7 \times 10^6$.

3. The production method for a product according to claim 1,
   wherein the cumulative number X [particles/mm$^2$] on Day 10 satisfies $0 < X \leq 1.3 \times 10^6$.

4. The production method for a product according to claim 1,
   wherein a maximum cumulative number X [particles/mm$^2$] in a culture period after Day 10 satisfies $0 < X \leq 1.0 \times 10^8$.

5. The production method for a product according to claim 1,
   wherein a maximum cumulative number X [particles/mm$^2$] in a culture period after Day 10 satisfies $0 < X \leq 1.5 \times 10^6$.

6. The production method for a product according to claim 1,
   wherein a minimum cumulative number X [particles/mm$^2$] in a culture period after Day 10 satisfies $0 < X \leq 1.0 \times 10^8$.

7. The production method for a product according to claim 1,
wherein a minimum cumulative number X [particles/mm$^2$] in a culture period after Day 10 satisfies $0 < X \leq 0.9 \times 10^6$.

8. The production method for a product according to any one of claims 1 to 7,
wherein a relationship between the cumulative number X [particles/mm$^2$] and the aeration rate V [vvm] satisfies $-10^4 \leq V - 10^5 \times X^{-0.9} \leq 0.1$.

9. The production method for a product according to any one of claims 1 to 7,
wherein a maximum shear rate D [1/s] applied to cells in a tube assembly from the culture tank to the membrane and in the membrane satisfies $0 < D \leq 65,000$.

10. The production method for a product according to claim 9,
wherein the maximum shear rate D [1/s] satisfies $0 < D \leq 39,000$.

11. The production method for a product according to claim 9,
wherein the maximum shear rate D [1/s] satisfies $0 < D \leq 3,000$.

12. The production method for a product according to any one of claims 1 to 7,
wherein a ratio [m$^2$/L] of a membrane area [m$^2$] per liquid amount [L] of the culture solution is 0.015 or more and 1.0 or less.

13. The production method for a product according to any one of claims 1 to 7,
wherein a time frequency T [s/day] at which a maximum shear rate is applied to cells in a tube assembly from the culture tank to the membrane and in the membrane satisfies $0 < T \leq 10,000$.

14. The production method for a product according to any one of claims 1 to 7,
wherein a cumulative time frequency $T_{total}$ [s/day] at which a shear rate of 100 [1/s] or more and 100,000 [1/s] or less is applied to cells in a tube assembly from the culture tank to the membrane and in the membrane satisfies $0 < T_{total} \leq 100,000$.

15. The production method for a product according to any one of claims 1 to 7,
wherein a relationship between a minimum inner diameter d [mm] of a tube and a joint of a tube assembly from the culture tank to the membrane and an amount C [L] of the culture solution satisfies $0.0001 \leq d/C \leq 100$.

16. The production method for a product according to any one of claims 1 to 7,
wherein a relationship between a length L [mm] of a flow channel, which is a minimum inner diameter d [mm], and an amount C [L] of the culture solution in a tube and a joint of a tube assembly from the culture tank to the membrane satisfies $0.001 \leq L/C \leq 1,000$.

17. The production method for a product according to any one of claims 1 to 7,
wherein a concentration [U/L] of a lactate dehydrogenase in the culture solution at the time of the production of the product is more than 0 and 100,000 or less.

18. The production method for a product according to any one of claims 1 to 7,
wherein a period of culturing the cells is 10 days or more and 100 days or less.

19. The production method for a product according to any one of claims 1 to 7,
wherein a period of culturing the cells is 20 days or more and 90 days or less.

20. The production method for a product according to any one of claims 1 to 7,
wherein a sparger pore diameter aerated in the culture solution is 1 to 100 $\mu$m.

21. The production method for a product according to any one of claims 1 to 7,
wherein a flux Y [LMH] during filtration satisfies $0 < Y \leq 10$.

22. The production method for a product according to any one of claims 1 to 7,
wherein the cell is an animal cell.

23. The production method for a product according to any one of claims 1 to 7, wherein the cell is a CHO cell.

24. The production method for a product according to any one of claims 1 to 7, wherein the product is an antibody.

# FIG. 1

## INTERNATIONAL SEARCH REPORT

| International application No. |
|---|
| **PCT/JP2023/035594** |

| A. | CLASSIFICATION OF SUBJECT MATTER |
|---|---|

*C12P 21/00*(2006.01)i; *C12P 21/08*(2006.01)i
FI:    C12P21/00 B; C12P21/08

According to International Patent Classification (IPC) or to both national classification and IPC

| B. | FIELDS SEARCHED |
|---|---|

Minimum documentation searched (classification system followed by classification symbols)

C12P21/00; C12P21/08

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | WO 2019/181234 A1 (FUJIFILM CORP) 26 September 2019 (2019-09-26) claims, examples | 1-24 |
| A | WO 2018/159847 A1 (FUJIFILM CORP) 07 September 2018 (2018-09-07) claims, examples | 1-24 |
| A | US 2019/0085284 A1 (CODIAK BIOSCIENCES, INC.) 21 March 2019 (2019-03-21) claims, examples | 1-24 |
| E, A | WO 2023/190829 A1 (FUJIFILM CORP) 05 October 2023 (2023-10-05) paragraph [0098], fig. 3 | 1-24 |

☐ Further documents are listed in the continuation of Box C.        ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **06 December 2023** | **19 December 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT
### Information on patent family members

International application No.

**PCT/JP2023/035594**

| Patent document cited in search report | | | Publication date (day/month/year) | Patent family member(s) | | | Publication date (day/month/year) |
|---|---|---|---|---|---|---|---|
| WO | 2019/181234 | A1 | 26 September 2019 | US | 2020/0399585 | A1 | |
| | | | | claims, examples | | | |
| | | | | EP | 3770266 | A1 | |
| | | | | CN | 111868249 | A | |
| WO | 2018/159847 | A1 | 07 September 2018 | US | 2019/0322975 | A1 | |
| | | | | claims, examples | | | |
| | | | | EP | 3591030 | A1 | |
| | | | | CN | 110268043 | A | |
| US | 2019/0085284 | A1 | 21 March 2019 | EP | 3684381 | A1 | |
| WO | 2023/190829 | A1 | 05 October 2023 | (Family: none) | | | |

Form PCT/ISA/210 (patent family annex) (January 2015)

**REFERENCES CITED IN THE DESCRIPTION**

**Patent documents cited in the description**

- WO 2019181234 A
- JP 2016517691 A